# EUROPÄISCHE PATENTSCHRIFT

(11) **EP 0 570 779 B1**
(45) Veröffentlichungstag und Bekanntmachung des Hinweises auf die Patenterteilung: **17.12.1997**
(21) Anmeldenummer: 93107418.1
(22) Anmeldetag: 07.05.1993
(51) Int. Cl.: D06M 13/419, C07C 233/18, D06P 1/649

(54) **Verwendung von tertiären Amiden als schaumarme Netzmittel in der Textilindustrie**
Use of tertiary amides as low foaming wetting agents in the textile industry
Utilisation d'amides tertiaires comme agent mouillant pauvre en mousse dans l'industrie textile

(30) Priorität: 16.05.1992 DE 4216316
(43) Veröffentlichungstag der Anmeldung: 24.11.1993
(73) Patentinhaber: BASF Aktiengesellschaft, 67063 Ludwigshafen (DE)
(72) Erfinder: Beckmann, Eberhard, Dr., W-6730 Neustadt (DE); Brueckmann, Ralf, Dr., Charlotte, N.C. 28270 (US); Dix, Johannes Peter, Dr., W-6179 Weisenheim (DE); Freyberg, Peter, Dr., W-6700 Ludwigshafen (DE); Kromm, Erich, Dr., W-6714 Weisenheim (DE)

(56) Entgegenhaltungen:
- EP-A- 0 258 500
- GB-A- 872 099
- GB-A- 2 169 961

## Beschreibung

Die vorliegende Erfindung betrifft die Verwendung von tertiären Amiden als schaumarme Netzmittel in der Textilindustrie.

Da ein Teil dieser tertiären Amide neue Stoffe darstellt, betrifft die Erfindung weiterhin diese neuen Stoffe.

In der Textilfärberei werden Netzmittel im Vorbereitungsprozeß und beim Vorbehandeln und Färben der Rohware benötigt. Das Netzen hat den Zweck, der Ware die Saugfähigkeit zu verleihen, die für alle nachfolgenden Naßprozesse erforderlich ist.

In der Literaturstelle J. Am. Oil Chem. Soc., Bd. 63, Nr. 12, Dezember 1986, S. 1579-1583 werden tertiäre Amide der Formel 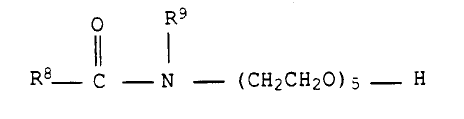 in der R⁸ und R⁹ jeweils für aliphatische Alkylreste mit einer Kettenlänge von 7 bis 10 C-Atomen, insbesondere 8 C-Atomen, stehen, als Netzmittel für Textilien empfohlen. Diese Verbindungen zeigen jedoch in ihren anwendungstechnischen Eigenschaften starke Mängel, insbesondere sind die Netzzeiten zu lang und die Schaumbildung ist zu stark, ferner neigen die Verbindungen zu Ausfällungen in den Anwendungsbädern.

Aus der U.S. Published Patent Application B 468 198 sind tertiäre Amide von C₈- bis C₂₀-Alkan- und -Alkensäuren, beispielsweise der Octansäure oder der Decansäure, die als Substituenten am Amidstickstoff einen Polyoxyalkylenrest und einen C₁- bis C₅-Alkylrest tragen, als Hilfsmittel bei der Polymerisation von Chloropren bekannt.

Die GB-A-2 169 916 betrifft eine Waschmittelformulierung, die als eine oberflächenaktive Komponente ein tertiäres Amid einer C₉- bis C₂₁-Alkansäure, insbesondere einer C₁₂- bis C₁₆-Fettsäure, welches als Substituenten am Amidstickstoff einen Polyoxyethylenrest und einen Alkylrest mit bis zu 3 C-Atomen trägt, enthält.

Aufgabe der vorliegenden Erfindung war es, Netzmittel für die Textilindustrie zur Verfügung zu stellen, die die geschilderten Mängel des Standes der Technik nicht mehr aufweisen.

Demgemäß wurde die Verwendung von tertiären Amiden der allgemeinen Formel I 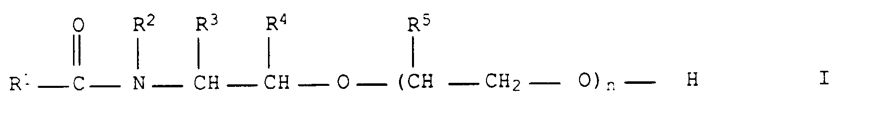 in der
- R¹: einen geradkettigen oder verzweigten C₅- bis C₁₇-Al-kyl- oder -Alkenylrest bezeichnet,
- R²: einen geradkettigen oder verzweigten C₃- bis C₆-Alkylrest bedeutet,
- R³ bis R⁵: unabhängig voneinander Wasserstoff, Methyl oder Ethyl bezeichnen und
- n: für eine Zahl von 3 bis 15 steht,
als schaumarme Netzmittel bei der Herstellung und Veredlung von Textilien gefunden.

Der Rest R¹ bezeichnet vorzugsweise geradkettiges oder verzweigtes C₆- bis C₁₅-Alkyl, insbesondere C₇- bis C₁₃-Alkyl. Beispiele für R¹ sind n-Pentyl, n-Hexyl, n-Heptyl, Hept-3-yl (abgeleitet von der 2-Ethylhexansäure), n-Octyl, 2,4,4-Trimethylpentyl (abgeleitet von der Hauptkomponente der Isononansäure), n-Nonyl, n-Decyl, n-Undecyl, n-Dodecyl, n-Tridecyl, n-Tetradecyl, n-Pentadecyl, n-Hexadecyl, n-Heptadecyl (abgeleitet von der Stearinsäure) oder cis-8-Heptadecenyl (abgeleitet von der Ölsäure). Besonders gute Ergebnisse als Netzmittel erhält man, wenn der Rest R¹ aus der Nonansäure oder insbesondere der Isononansäure stammt.

Der Rest R² bedeutet vorzugsweise geradkettiges oder verzweigtes C₄- bis C₆-Alkyl, insbesondere geradkettiges Alkyl wie n-Butyl, n-Pentyl oder n-Hexyl. Daneben kann R² aber auch n-Propyl, iso-Propyl, sec.-Butyl, iso-Butyl, tert.-Butyl, sec.-Pentyl, iso-Pentyl, tert.-Pentyl, neo-Pentyl, 2-, 3- oder 4-Methylpentyl, 2,3- oder 3,3-Dimethylbutyl bedeuten. Die Reste R³ bis R⁵ bezeichnen vorzugsweise Wasserstoff.

Die Variable n steht vorzugsweise für eine Zahl von 5 bis 10.

Die tertiären Amide I sind nach im Prinzip bekannten Methoden herstellbar. Zweckmäßigerweise werden hierzu Amide der allgemeinen Formel II 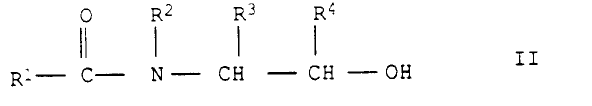 in der die Variablen R¹ bis R⁴ die oben genannten Bedeutungen haben, mit einem C₂- bis C₄-Alkylenoxid wie Propylenoxid, Butylenoxid oder insbesondere Ethylenoxid in üblicher Weise umgesetzt. Man arbeitet hierbei in der Regel unter erhöhtem Druck bei etwa 2 bis 15 bar, insbesondere 4 bis 10 bar, bei Temperaturen von etwa 80 bis 170°C, insbesondere 100 bis 150°C. Als Oxalkylierungskatalysatoren können alle hierfür üblichen Substanzen verwendet werden, besonders gut eignet sich Kalium-tert.-butylat. Die Oxalkylierungsprodukte werden wie üblich aufgearbeitet, nicht umgesetztes Alkylenoxid wird normalerweise im Vakuum abgezogen.

Die Amide II sind beispielsweise aus den entsprechenden Carbonsäuren der allgemeinen Formel III 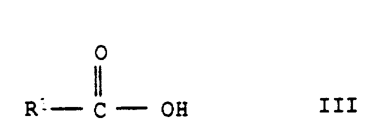 und den entsprechenden sekundären Aminen der allgemeinen Formel IV 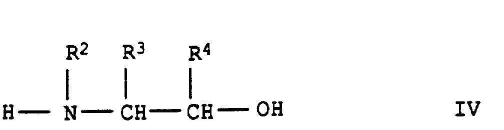 leicht durch Umsetzung bei erhöhter Temperatur erhältlich.

Die erfindungsgemäß verwendeten tertiären Amide I eignen sich in hervorragender Weise als schaumarme Netzmittel bei der Herstellung und Veredlung von Textilien, insbesondere in der Textilfärberei bei der Vorbereitung, der Vorbehandlung und dem Färbeprozeß von Rohware. Als textile Materialien kommen hierbei insbesondere Baumwolle und Baumwoll-Mischgewebe in Betracht.

Neben der Schaumarmut sind die geringen Netzzeiten der Verbindungen I besonders vorteilhaft. Die Verbindungen I neigen außerdem nicht zu Ausfällungen in den Anwendungsbädern.

Ein Teil der tertiären Amide I stellt neue Stoffe dar. Deshalb betrifft die Erfindung weiterhin tertiäre Amide der allgeinen Formel Ia 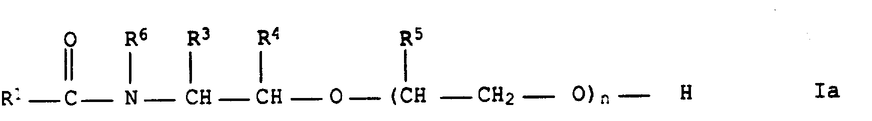 in der
- R¹: einen geradkettigen oder verzweigten C₅- bis C₁₇-Alkyl- oder -Alkenylrest bezeichnet,
- R³ bis R⁵: unabhängig voneinander Wasserstoff, Methyl oder Ethyl bezeichnen,
- R⁶: n-Butyl, n-Pentyl oder einen geradkettigen oder verzweigten C₆-Alkylrest bedeuten und
- n: für eine Zahl von 3 bis 15 steht.

Weiterhin betrifft die Erfindung auch tertiäre Amide der allgemeinen Form Ib 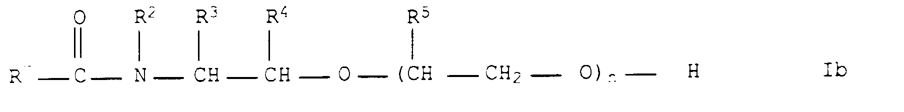 in der
- R²: einen geradkettigen oder verzweigten C₃- bis C₆-Alkylrest bedeutet,
- R³ bis R⁵: unabhängig voneinander Wasserstoff, Methyl oder Ethyl bezeichnen,
- R: den Rest der Nonansäure oder der Isononansäure bedeutet und
- n: für eine Zahl von 3 bis 5 steht.

### Herstellungsbeispiele

### Beispiel 1

Zu 158,2 g (1,0 mol) Nonansäure (Pelargonsäure) wurden bei Umgebungstemperatur 175,8 g (1,5 mol) N-Butylethanolamin unter Schutzgasatmosphäre zugegeben. Anschließend wurde 2 h bei 140°C, 6 h bei 150°C und 8 h bei 160°C nachgerührt. Um das überschüssige N-Butylethanolamin zu entfernen, wurde für die Dauer von 2 h ein Vakuum von 0,1 mbar angelegt. Man erhielt 250 g (entsprechend einer Ausbeute von 91 %) Nonansäure-N-butyl-N-(2-hydroxyethyl)-amid.

128,7 g (0,5 mol) dieses Amids wurden in einem Autoklaven mit 1,3 g Kalium-tert.-butylat versetzt und bei 110 bis 120°C portionsweise mit 165 g (3,75 mol) Ethylenoxid bei einem Druck von 5 bis 10 bar umgesetzt. Nach Beendigung der Ethoxylierung wurde geringe Mengen nicht umgesetzten Ethylenoxids während 1 h bei 80°C/l mbar abgezogen. Man erhielt 290 g mit 7,5 mol Ethylenoxid umgesetztes Nonansäure-N-butyl-N-(2-hydroxyethyl)amid.

### Beispiel 2

In Analogie zu Beispiel 1 wurde ausgehend von Isononansäure und N-Butylethanolamin in der ersten Stufe und 10 mol Ethylenoxid pro Mol Zwischenprodukt in der zweiten Stufe mit 10 mol Ethylenoxid umgesetztes Isononansäure-N-butyl-N-(2-hydroxyethyl)amid hergestellt.

### Beispiel 3

In Analogie zu Beispiel 1 wurde ausgehend von Isononansäure und N-Hexylethanolamin in der ersten Stufe und 8 mol Ethylenoxid pro Mol Zwischenprodukt in der zweiten Stufe mit 8 mol Ethylenoxid umgesetztes Isononansäure-N-hexyl-N-(2-hydroxyethyl)amid hergestellt.

### Beispiel 4

In Analogie zu Beispiel 1 wurde ausgehend von Isononansäure und N-Hexylisopropanolamin in der ersten Stufe und 9 mol Ethylenoxid pro Mol Zwischenprodukt in der zweiten Stufe mit 9 mol Ethylenoxid umgesetzes Isononansäure-N-hexyl-N-(2-hydroxypropyl)amid hergestellt.

### Vergleichsbeispiel A

In Analogie zu Beispiel 1 wurde ausgehend von Nonansäure und N-(2-Ethylhexyl)-ethanolamin in der ersten Stufe und 10 mol Ethylenoxid pro Mol Zwischenprodukt in der zweiten Stufe mit 10 mol Ethylenoxid umgesetztes Nonansäure-N-(2-ethylhexyl) -N- (2-hydroxyethyl)amid hergestellt.

### Vergleichsbeispiel B

In Analogie zu Beispiel 1 wurde ausgehend von Isonansäure und N-(2-Ethylhexyl)ethanolamin in der ersten Stufe und 10 mol Ethylenoxid pro Mol Zwischenprodukt in der zweiten Stufe mit 10 mol Ethylenoxid umgesetztes IsononansäureN-(2-ethylhexyl)-N-(2-hydroxyethyl)amid hergestellt.

### Anwendungstechnische Beispiele

Das Netzvermögen wurde nach der Tauchnetzmethode gemäß DIN 53901 bestimmt. Bei diesem Verfahren wird mit einer Tauchklemme ein standardisiertes Stück Baumwoll-Gewebe in eine wäßrige Tensidlösung eingebracht. Durch das Benetzen dringt Flüssigkeit in das Gewebe, wodurch die darin enthaltene Luft verdrängt und der Auftrieb kompensiert wird. Das Gewebe sinkt dann zu Boden. Gemessen wird die Zeit zwischen Eintauchen und Beginn des Absinkens.

In Tabelle 1 sind die Netzzeiten der 0,2 gew.-%igen neutralen Substanzlösungen angegeben.

**Tabelle 1**

| Substanz aus Bsp. Nr. | Netzzeit [sec] | |
|---|---|---|
| | bei 25°C | bei 70°C |
| erfindungsgemäß: | | |
| 1 | 48 | - |
| 2 | 29 | 40 |
| 3 | 22 | 22 |
| 4 | 15 | 34 |
| zum Vergleich: | | |
| A | 11 | Ausfällung |
| B | 12 | Ausfällung |

Das Schaumvermögen wurde nach der modifizierten Schlagschaum-Methode gemäß DIN 53 902 bestimmt. Dazu wird eine wäßrige Tensidlösung in einen Stand-Meßzylinder eingebracht und durch Schlagen der Lösung mit einer an einem Stiel befestigten Lochscheibe Schaum erzeugt. Bestimmt werden die Schaumvolumina nach 30, 60 sowie nach 120 Sekunden.

Tabelle 2 zeigt das Schaumvermögen der 0,2 gew.%igen neutralen Substanzlösungen.

**Tabelle 2**

| Substanz aus Bsp. | Schaumvolumen [ml] | |
|---|---|---|
| Nr. | bei 25°C | bei 70°C |
| erfindungsgemäß: | | |
| 1 | 10/10/10 | 0/0/0 |
| 2 | 10/10/0 | 10/10/10 |
| 3 | 50/40/30 | 0/0/0 |
| 4 | 120/70/50 | 10/0/0 |
| zum Vergleich: | | |
| A | 460/400/360 | 300/210/80 |
| B | 90/40/20 | 20/10/10 |

## Patentansprüche

1. Verwendung von tertiären Amiden der allgemeinen Formel I 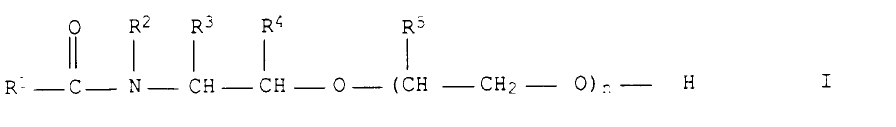 in der
R¹ einen geradkettigen oder verzweigten C₅- bis C₁₇-Alkyl- oder -Alkenylrest bezeichnet,
R² einen geradkettigen oder verzweigten C₃- bis C₆-Alkylrest bedeutet,
R³ bis R⁵ unabhängig voneinander Wasserstoff, Methyl oder Ethyl bezeichnen und
n für eine Zahl von 3 bis 15 steht,
als schaumarme Netzmittel bei der Herstellung und Veredlung von Textilien.

2. Verwendung von tertiären Amiden I nach Anspruch 1, bei denen R¹ einen geradkettigen oder verzweigten C₆- bis C₁₅-Alkylrest bezeichnet.

3. Verwendung von tertiären Amiden I nach Anspruch 1 oder 2, bei denen R² einen geradkettigen oder verzweigten C₄- bis C₆-Alkylrest bedeutet.

4. Verwendung von tertiären Amiden I nach den Ansprüchen 1 bis 3, bei denen R³ bis R⁵ Wasserstoff bezeichnen.

5. Verwendung von tertiären Amiden I nach den Ansprüchen 1 bis 4, bei denen n für eine Zahl von 5 bis 10 steht.

6. Verfahren zur Herstellung und zur Veredlung von Textilien, dadurch gekennzeichnet, daß man hierbei tertiäre Amide I gemäß den Ansprüchen 1 bis 5 als schaumarme Netzmittel verwendet.

7. Tertiäre Amide der allgemeinen Formel Ia 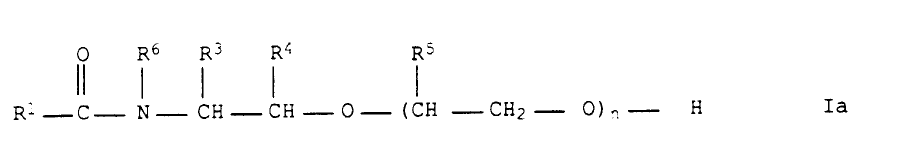 in der
R¹ einen geradkettigen oder verzweigten C₅- bis C₁₇-Alkyl- oder -Alkenylrest bezeichnet,
R³ bis R⁵ unabhängig voneinander Wasserstoff, Methyl oder Ethyl bezeichnen,
R⁶ n-Butyl, n-Pentyl oder einen geradkettigen oder verzweigten C₆-Alkylrest bedeuten und
n für eine Zahl von 3 bis 5 steht.

8. Tertiäre Amide der allgemeinen Formel Ib 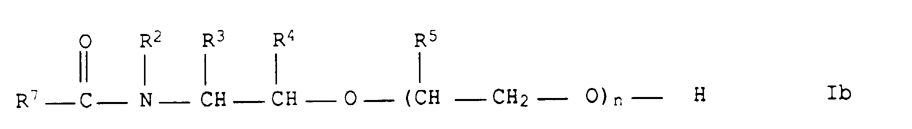 in der
R² einen geradkettigen oder verzweigten C₃- bis C₆-Alkylrest bedeutet,
R³ bis R⁵ unabhängig voneinander Wasserstoff, Methyl oder Ethyl bezeichnen und
R⁷ den Rest der Nonansäure oder der Isononansäure bedeutet und
n für eine Zahl von 3 bis 5 steht.

## Claims

1. The use of tertiary amides of the general formula I 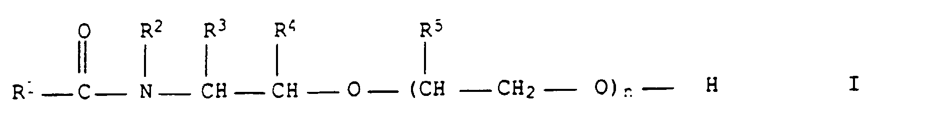 where
R¹ is straight-chain or branched C₅-C₁₇-alkyl or -alkenyl,
R² is straight-chain or branched C₃-C₆-alkyl,
R³, R⁴ and R⁵ are each independently of the others hydrogen, methyl or ethyl, and
n is from 3 to 15,
as low-foam wetting agents in the manufacture and finishing of textiles.

2. The use as claimed in claim 1 of tertiary amides I where R¹ is straight-chain or branched C₆-C₁₅-alkyl.

3. The use as claimed in claim 1 or 2 of tertiary amides I where R² is straight-chain or branched C₄-C₆-alkyl.

4. The use as claimed in any of claims 1 to 3 of tertiary amides I where R³, R⁴ and R⁵ are each hydrogen.

5. The use as claimed in any of claims 1 to 4 of tertiary amides I where n is from 5 to 10.

6. A process for manufacturing and finishing textiles, characterized in that it comprises using tertiary amides I as claimed in any of claims 1 to 5 as low-foam wetting agents.

7. Tertiary amides of the general formula Ia 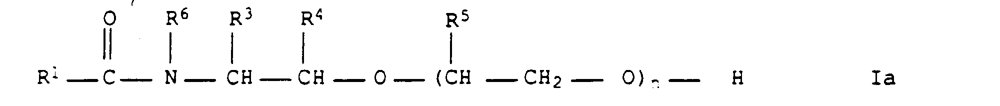 where
R¹ is straight-chain or branched C₅-C₁₇-alkyl or -alkenyl,
R³, R⁴ and R⁵ are each independently of the others hydrogen, methyl or ethyl,
R⁶ is n-butyl, n-pentyl or straight-chain or branched C₆-alkyl, and
n is from 3 to 5.

8. Tertiary amides of the general formula Ib 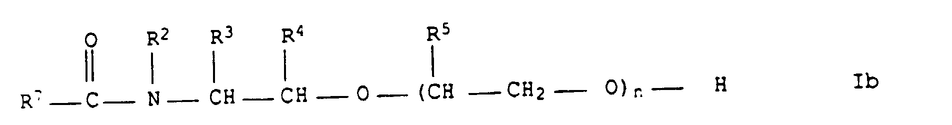 where
R² is straight-chain or branched C₃-C₆-alkyl,
R³, R⁴ and R⁵ are each independently of the others hydrogen, methyl or ethyl,
R⁷ is the radical of nonanoic acid or isononanoic acid, and
n is from 3 to 5.

## Revendications

1. Utilisation d'amides tertiaires de formule générale I 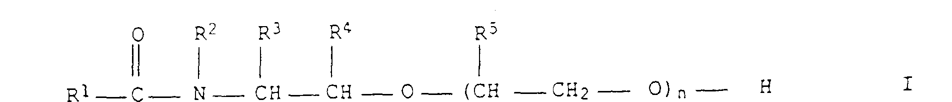 dans laquelle
R¹ représente un reste alcényle ou alkyle en C₅-C₁₇ linéaire ou ramifié,
R² représente un reste alkyle en C₃-C₆ linéaire ou ramifié,
R³ à R⁵ représentent, indépendamment les uns des autres, des atomes d'hydrogène, des groupements méthyle ou éthyle,
n est mis pour un nombre de 3 à 15,
en tant qu'agent mouillant pauvre en mousse pour la fabrication et la finition de textiles.

2. Utilisation d'amides tertiaires I selon la revendication 1, pour lesquels R¹ représente un groupement alkyle en C₆-C₁₅ linéaire ou ramifié.

3. Utilisation d'amides tertiaires I selon la revendication 1 ou 2, pour lesquels R² représente un reste alkyle en C₄-C₆ linéaire ou ramifié.

4. Utilisation d'amides tertiaires I selon l'une quelconque des revendications 1 à 3, pour lesquels R³ à R⁵ représentent des atomes d'hydrogène.

5. Utilisation d'amides tertiaires I selon l'une quelconque des revendications 1 à 4, pour lesquels n est mis pour un nombre de 5 à 10.

6. Procédé de préparation et de finition de textiles, caractérisé en ce que l'on utilise des amides tertiaires I selon l'une quelconque des revendications 1 à 5 en tant qu'agent mouillant pauvre en mousse.

7. Amides tertiaires de formule générale Ia 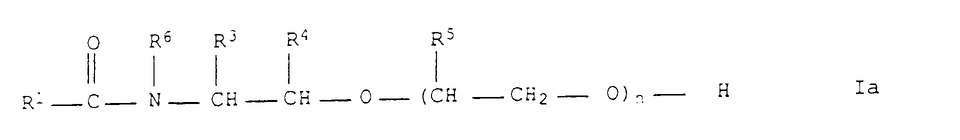 dans laquelle
R¹ représente un reste alcényle ou alkyle en C₅-C₁₇ linéaire ou ramifié,
R³ à R⁵ représentent, indépendamment les uns des autres, des atomes d'hydrogène, des groupements méthyle ou éthyle,
R⁶ représente un groupement n-butyle, n-pentyle ou un groupement alkyle en C₆ linéaire ou ramifié, et
n est mis pour un nombre de 3 à 5,

8. Amides tertiaires de formule générale Ib 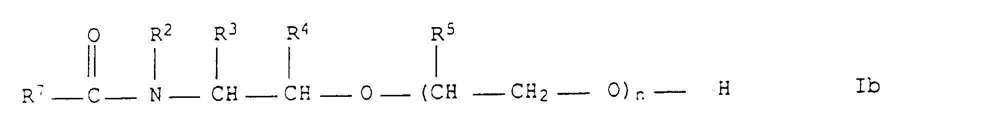 dans laquelle
R² représente un reste alkyle en C₃-C₆ linéaire ou ramifié,
R³ à R⁵ représentent, indépendamment les uns des autres, des atomes d'hydrogène, des groupements méthyle ou éthyle,
R⁷ représente un reste d'acide nonanoïque ou isononanoïque et
n est mis pour un nombre de 3 à 5.
